# EUROPÄISCHE PATENTSCHRIFT

(11) **EP 2 779 985 B1**
(45) Veröffentlichungstag und Bekanntmachung des Hinweises auf die Patenterteilung: **19.12.2018**
(21) Anmeldenummer: 12778114.4
(22) Anmeldetag: 25.10.2012
(51) Int. Cl.: A61K 8/41, A61K 8/49, A61K 8/81, A61Q 5/12

(54) **PFLEGENDE HAARBEHANDLUNGSMITTEL**
HAIR CARE COMPOSITIONS
PRODUITS DE SOINS CAPILLAIRES

(30) Priorität: 17.11.2011 DE 102011086537
(43) Veröffentlichungstag der Anmeldung: 24.09.2014
(73) Patentinhaber: Henkel AG & Co. KGaA, 40589 Düsseldorf (DE)
(72) Erfinder: VON ASPERN, Edith, 21271 Hanstedt (DE); DELOWSKY, Jens, 22844 Norderstedt (DE); KAHRE, Jörg, 42799 Leichlingen (DE)
(86) Internationale Anmeldenummer: PCT/EP2012/071122
(87) Internationale Veröffentlichungsnummer: WO 2013/072171

(56) Entgegenhaltungen:
- EP-A2- 2 335 671
- WO-A2-2009/074464
- WO-A2-2010/000645
- WO-A2-2011/000698
- Anonymous: "TETRANYL, QUARTAMIN and AKYPO QUAT", , XP002724519, Gefunden im Internet: URL:http://p31409.typo3server.info/fileadm in/KAO%20uploads/01_Personal_Care/011_Trad e_Name/quartamin_tetranyl_akypoquat.pdf [gefunden am 2014-05-19]
- ANONYMOUS: "Ciba Launches Cationic Rheology Modifier", INTERNET CITATION, 15. April 2008 (2008-04-15), Seiten 1-3, XP002631019, Gefunden im Internet: URL:http://www.happi.com/news/2008/04/15/c iba_launches_cationic_rheology_modifier [gefunden am 2011-03-31]
- "Personal Care Compositions with Beneficial Properties", IP.COM JOURNAL, IP.COM INC., WEST HENRIETTA, NY, US, 3. April 2009 (2009-04-03), XP013130819, ISSN: 1533-0001

## Beschreibung

### "Pflegende Haarbehandlungsmittel"

Die Erfindung betrifft kosmetische Haarbehandlungsmittel auf der Basis einer speziellen Wirkstoffkombination.

Nicht zuletzt durch die starke Beanspruchung der Haare, beispielsweise durch das Färben oder Dauerwellen als auch durch die Reinigung der Haare mit Shampoos und durch Umweltbelastungen, nimmt die Bedeutung von Pflegeprodukten mit möglichst langanhaltender Wirkung zu.

Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften und guter biologischer Abbaubarkeit. Insbesondere in farbstoff- und/oder elektrolythaltigen Formulierungen besteht Bedarf an zusätzlichen pflegenden Wirkstoffen, die sich problemlos in bekannte Formulierungen einarbeiten lassen.

Haarbehandlungsmittel, die ein oder mehrere quartäre Ammoniumverbindungen (QAV) als Pflegestoffe enthalten, sind im Stand der Technik breit bekannt.

In WO 2010/00645 werden Haarkuren offenbart, die als Pflegestoffe neben quartären Ammoniumverbindungen Polyquaternium-11 enthalten. Diese sind hinsichtlich ihrer Haarpflegewirkung verbesserungsfähig.

Der vorliegenden Erfindung lag die Aufgabe zugrunde, diese Wirkstoffkombinationen weiterzuentwickeln und insbesondere im Hinblick auf die Kämmbarkeit und Weichheit (Griff) keratinischer Fasern zu verbessern. Darüber hinaus bestand die Aufgabe der vorliegenden Erfindung darin, eine Wirkstoffkombination bereitzustellen, die bei insgesamt verringerten Einsatzmengen hervorragende Pflegeleistungen liefert.

Es wurde nun gefunden, dass der Zusatz bestimmter Polymere zu einer bestimmten Kombination von drei verschiedenen QAV die Kämmbarkeiten, den Glanz und die Elastizität noch weiter verbessert. Darüber hinaus konnte auch die Waschbeständigkeit gefärbten Haares verbessert werden. Überraschenderweise wurde insbesondere der Griff der keratinischen Fasern außerordentlich positiv beeinflusst. Die genannten Effekte traten dabei überraschenderweise auch bei insgesamt reduzierter Gesamtmenge an kationischen Aktivstoffen deutlich zutage.

Gegenstand der vorliegenden Erfindung sind in einer ersten Ausführungsform Haarbehandlungsmittel, enthaltend
a) mindestens eine quartäre Ammoniumverbindung (QAV) gemäß der Formel I 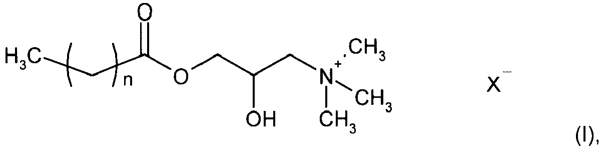 in der n für eine ganze Zahl von 10 bis 24 und X⁻ für ein Anion steht,
b) mindestens eine quartäre Ammoniumverbindung (QAV) gemäß der Formel II 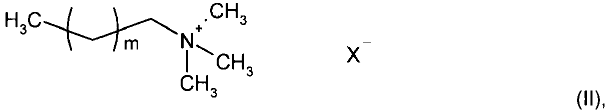 in der m für eine ganze Zahl von 10 bis 24 und X⁻ für ein Anion steht,
c) mindestens eine quartäre Ammoniumverbindung (QAV) der Formel III 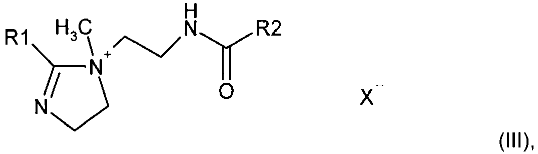 in der R1 und R2 unabhängig voneinander jeweils für einen gesättigten oder ungesättigten, linearen oder verzweigten Kohlenwasserstoffrest mit einer Kettenlänge von 18 bis 30 Kohlenstoffatomen stehen und X⁻ für ein Anion steht,
d) mindestens ein kationisches Copolymer, enthaltend Monomere der Formel (IV) und Monomere der Formel (V) 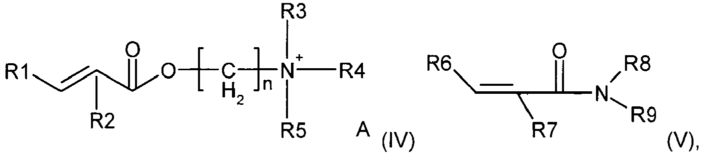 in denen R¹ bis R⁹ unabhängig voneinander Wasserstoff, C₁₋₄-Alkyl wie Methyl, Ethyl, Propyl, iso-Propyl, Butyl, iso-Butyl oder tert-Butyl bedeuten mit der Maßgabe, dass mindestens einer der Reste R⁶, R⁷, R⁸ oder R⁹ C₁₋₄-Alkyl bedeutet, n für ganze Zahlen von 1 bis 8, und A- für ein physiologisch verträgliches Anion steht.

Haarbehandlungsmittel im Sinne der vorliegenden Erfindung sind beispielsweise Haarfärbemittel, Blondiermittel, Haarshampoos, Haarkonditionierer, konditionierende Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen. Bevorzugte erfindungsgemäße Mittel sind Shampoos, Konditioniermittel oder Haar-Tonics.

Als Inhaltstoff a) enthalten die erfindungsgemäßen Mittel mindestens eine quartäre gemäß der Formel I 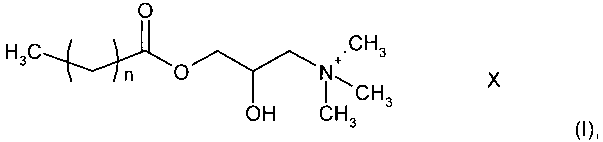 in der n für eine ganze Zahl von 10 bis 24 und X- für ein Anion steht. Verbindungen der Formel (I) werden in der INCI-Nomenklatur als Alkanoyl PG-Trimoniumsalze bezeichnet. Bevorzugte Werte für n sind die Zahlen 16, 18 und 20, bevorzugte Anionen sind Chlorid und Methosulfat. Die QAV der Formel (I) sind in den erfindungsgemäßen Zusammensetzungen vorzugsweise in Mengen von 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten. Im Hinblick auf die Lagerstabilität der erfindungsgemäßen Zusammensetzungen sind erfindungsgemäße Haarbehandlungsmittel besonders bevorzugt, die - bezogen auf ihr Gewicht - 0,1 bis 20 Gew.-%, vorzugsweise 0,2 bis 10 Gew.-%, weiter bevorzugt 0,3 bis 7,5 Gew.-%, noch weiter bevorzugt 0,4 bis 5 Gew.-% und insbesondere 0,5 bis 2,5 Gew.-% mindestens einer QAV der Formel I enthalten, in der n für die Zahl 20 und A⁻ für Chlorid steht (INCI-Bezeichnung: Behenoyl PG Trimonium Chloride).

Als Inhaltstoff b) enthalten die erfindungsgemäßen Mittel mindestens eine quartäre Ammoniumverbindung gemäß der Formel II

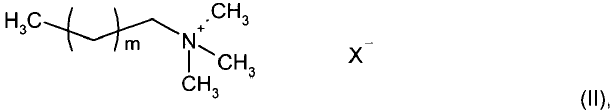

in der m für eine ganze Zahl von 10 bis 24 und X- für ein Anion steht. Verbindungen der Formel (II) werden in der INCl-Nomenklatur als Alkan-Trimoniumsalze bezeichnet. Bevorzugte Werte für n sind die Zahlen 16, 18 und 20, bevorzugte Anionionen sind Chlorid und Methosulfat.

Die QAV der Formel (II) sind in den erfindungsgemäßen Zusammensetzungen vorzugsweise in Mengen von 0,01 bis 20 Gew.-%, bevorzugt in Mengen von 0,01 bis 10 Gew.-% und ganz besonders bevorzugt in Mengen von 0,1 bis 7,5 Gew.-% enthalten. Die allerbesten Ergebnisse werden dabei mit Mengen von 0,1 bis 5 Gew.-% jeweils bezogen auf die Gesamtzusammensetzung des jeweiligen Mittels erhalten. Im Hinblick auf die Pflegeleistung, insbesondere die Entwirrbarkeit, Kämmbarkeit und den Griff der mit erfindungsgemäßen Zusammensetzungen behandelten Haare sind erfindungsgemäße Haarbehandlungsmittel bevorzugt, die - bezogen auf sein Gewicht - 0,1 bis 15 Gew.-%, vorzugsweise 0,2 bis 10 Gew.-%, weiter bevorzugt 0,3 bis 7,5 Gew.-%, noch weiter bevorzugt 0,4 bis 5 Gew.-% und insbesondere 0,5 bis 2,5 Gew.-% mindestens einer QAV der Formel II enthalten, in der n für die Zahl 20 und A- für Chlorid steht (INCl-Bezeichnung: Behentrimonium Chloride).

Als Inhaltstoff c) enthalten die erfindungsgemäßen Mittel mindestens eine QAV der Formel III 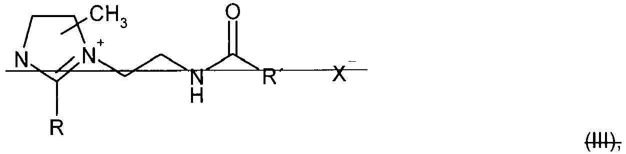 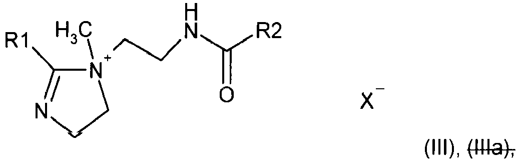 in der die Reste R¹ und R² unabhängig voneinander jeweils für einen gesättigten oder ungesättigten, linearen oder verzweigten Kohlenwasserstoffrest mit einer Kettenlänge von 16 bis 30 Kohlenstoffatomen stehen und X⁻ für ein Anion steht.

Vorzugsweise enthalten die erfindungsgemäßen Mittel Verbindungen der Formel (III), in der R¹ = R² gilt. Die Kettenlänge der Reste R beträgt mindestens 16 Kohlenstoffatome. Bevorzugt sind Verbindungen mit einer Kettenlänge von 16, 18 oder 20 Kohlenstoffatomen und besonders bevorzugt mit 16 oder 18 Kohlenstoffatomen. Eine ganz besonders bevorzugte Verbindung der Formel III weist als Reste R1 bzw. R2 Alkylreste auf, die sich vom Talgalkohol ableiten. Ein Handelsprodukt dieser Kettenlänge ist beispielsweise unter der Bezeichnung Quaternium-87 bekannt.

Das Gegenion X- in Formel (I) ist vorzugsweise Methosulfat, geeignet sind jedoch als Gegenionen auch die Halogenide wie Chlorid, Fluorid, Bromid. oder auch Phosphate.

Die Imidazoline der Formeln (III) sind in den erfindungsgemäßen Zusammensetzungen vorzugsweise in Mengen von 0,01 bis 20 Gew.-%, bevorzugt in Mengen von 0,01 bis 10 Gew.-% und ganz besonders bevorzugt in Mengen von 0,1 bis 7,5 Gew.-% enthalten. Die allerbesten Ergebnisse werden dabei mit Mengen von 0,1 bis 5 Gew.-% jeweils bezogen auf die Gesamtzusammensetzung des jeweiligen Mittels erhalten.

Ganz besonders bevorzugte erfindungsgemäße Haarbehandlungsmittel sind dadurch gekennzeichnet, dass sie - bezogen auf ihr Gewicht - 0,1 bis 15 Gew.-%, vorzugsweise 0,2 bis 10 Gew.-%, weiter bevorzugt 0,3 bis 7,5 Gew.-%, noch weiter bevorzugt 0,4 bis 5 Gew.-% und insbesondere 0,5 bis 2,5 Gew.-% mindestens einer QAV der Formel III enthalten, in der R und R' unabhängig voneinander ausgewählt sind aus Stearyl- und Oleoylresten und A⁻ für Methosulfat steht (INCl-Bezeichnung: Quaternium-87).

Als Inhaltstoff d) enthalten die erfindungsgemäßen Mittel mindestens ein kationisches Copolymer. Das kationische Copolymer ist hierbei aus den Monomeren der Formel (IV) und (V) aufgebaut, 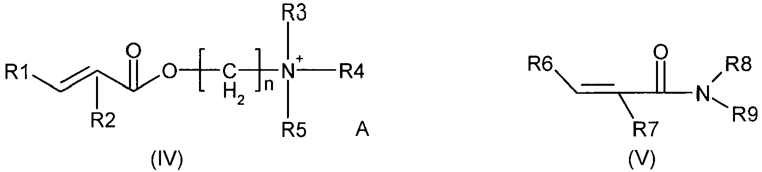 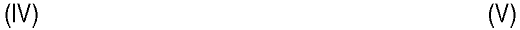 in welchen R¹ bis R⁹ unabhängig voneinander Wasserstoff, C₁-C₄-Alkyl wie Methyl-, Ethyl-, Propyl-, iso-Propyl-, Butyl-, iso-Butyl- oder tert-Butyl- bedeuten und der Maßgabe, dass mindestens einer der Reste R⁶, R⁷, R⁸ oder R⁹ C₁-C₄-Alkyl bedeutet, n ganzzahlig ist und für Zahlen von 1 bis 8 steht, und A⁻ für ein physiologisch verträgliches Anion wie Fluorid, Chlorid, Bromid, lodid, Hydrogensulfat oder Methosulfat steht.

Vorzugsweise ist R¹ Wasserstoff oder eine Methylgruppe, besonders bevorzugt ist R¹ eine Methylgruppe. Vorzugsweise ist R² Wasserstoff oder eine C₁-C₄-Alkylgruppe, besonders bevorzugt ist R² Wasserstoff. Vorzugsweise sind R³, R⁴ und R⁵ unabhängig voneinander Wasserstoff oder eine C₁-C₄-Alkylgruppe, insbesondere eine Methylgruppe. Besonders bevorzugt sind R³, R⁴ und R⁵ gleich und höchst bevorzugt Methyl. Die Zahl n ist bevorzugt ganzzahlig und steht für Zahlen von 1 bis 7, bevorzugter von 1 bis 5, höchst bevorzugt 1, 2, 3 oder 4 und insbesondere für 2. R⁶ ist bevorzugt Wasserstoff oder Methyl. R⁷ ist bevorzugt Wasserstoff, Methyl oder Ethyl, besonders bevorzugt Methyl. R⁸ und R⁹ sind bevorzugt gleich und stehen für Wasserstoff, Methyl oder C₁-C₄-Alkyl, besonders bevorzugt Wasserstoff oder Methyl. Am bevorzugtesten steht das Monomer der Formel (V) für Dimethylacrylamid.

Das kationische Copolymer d) enthält bevorzugt 20 bis 95 Gew.-% Monomere der Formel (IV) und 5 bis 50 Gew.-% Monomere der Formel (V).

Besonders bevorzugte kationische Copolymere d) enthalten 40 bis 90 Gew.-% Monomer (IV) und 10 bis 40 Gew.-% Monomer (V).

Das kationische Copolymer d) kann weiterhin vernetzt sein. In diesem Falle wird die Vernetzung durch die üblichen Vernetzungsmittel wie beispielsweise Allylacrylamid, Allylmethacrylamid, Tetraallylammoniumchlorid oder N,N'-methylen-bisacrylamid in Mengen bis zu 500 ppm bewirkt. Das kationische Copolymer d) kann weiterhin als eine Lösung, Suspension oder Dispersion in geeigneten kosmetischen Medien, wie beispielsweise Propylenglykol, Glycerin, Paraffin, Isoparaffin, Propylenglykolen und Estern von Propylenglykol wie beispielsweise Propylenglykol Dicaprylate/Dicaprate, oder PPG-1 Trideceth-6 und weiteren vorliegen.

Ein Beispiel für ein besonders bevorzugtes Copolymer d) ist das Handelsprodukt Tinovis® CD der Firma Ciba.

Das kationische Copolymer d) wird in den erfindungsgemäßen Mitteln bevorzugt in Mengen von 0,1 bis 15 Gew.-% und besonders bevorzugt in Mengen von 0,1 bis 5,0 Gew.-% eingesetzt - bezogen auf das Gewicht des Mittels.

Ganz besonders bevorzugte erfinddungsgemäße Haarbehandlungsmittel sind dadurch gekennzeichnet, dass sie - bezogen auf ihr Gewicht - 0,1 bis 15 Gew.-%, vorzugsweise 0,2 bis 10 Gew.-%, weiter bevorzugt 0,3 bis 7,5 Gew.-%, noch weiter bevorzugt 0,4 bis 5 Gew.-% und insbesondere 0,5 bis 2,5 Gew.-% mindestens eines kationischen Copolymers, enthaltend Monomere der Formel (IV) und Monomere der Formel (V), in denen R¹ = R⁶ = R⁷ = -H und R² = R³ = R⁴ = R⁵ = R⁸ = R⁹ = -CH₃ gilt, n für die Zahl 2 und A⁻ für Chlorid steht (INCI-Bezeichnung: Dimethylacrylamide/Ethyltrimonium Chloride Methacrylate Copolymer) enthalten.

Die in den erfindungsgemäßen Mitteln anhaltende Kombination von vier Aktivstoffen kann durch weitere Hilfsstoffe ergänzt werden. So hat sich beispielsweise gezeigt, dass Ester von langkettigen Alkoholen (Fettalkoholen) mit längerkettigen Monocarbonsäuren die Fülle und Weichheit des Haares noch weiter steigern, wenn sie in die erfindungsgemäßen Mittel eingearbeitet werden. Erfindungsgemäß besonders bevorzugte Haarbehandlungsmittel sind dadurch gekennzeichnet, dass sie zusätzlich Ester von Kokosfettalkohlen mit Octansäure enthalten.

Auch andere Emollients wie Dicaprylylcarbonat, Dicaprylylether, Propylheptylcaprylat usw. zeigen diese Effekte, sie sind jedoch bei den Kokosalkylestern der Octansäure besonders ausgeprägt. Ganz besonders bevorzugte erfindungsgemäße Mittel enthalten
- 0,1 bis 15 Gew.-% Behenoyl PG Trimonium Chloride und
- 0,1 bis 15 Gew.-% Behentrimonium Chloride und
- 0,1 bis 15 Gew.-% Quaternium-87 und
- 0,1 bis 15 Gew.-% Dimethylacrylamide/Ethyltrimonium Chloride Methacrylate Copolymer und
- 0,1 bis 10 Gew.-% Ester von Kokosfettalkohlen mit Octansäure.

Der Einsatz von Spreitmitteln hat sich ebenfalls als besonders vorteilhaft erwiesen, da die Pflegeeffekte der erfindungsgemäßen Kombination hierdurch noch gleichmäßiger zur Wirkung kommen. Besonders bevorzugte erfindungsgemäße Haarbehandlungsmittel sind dadurch gekennzeichnet, dass sie - bezogen auf ihr Gewicht - zusätzlich 0,1 bis 20 Gew.-%, vorzugsweise 0,2 bis 10 Gew.-%, weiter bevorzugt 0,3 bis 7,5 Gew.-%, noch weiter bevorzugt 0,4 bis 5 Gew.-% und insbesondere 0,5 bis 2,5 Gew.-% Isopropylmyristat enthalten.

Ganz besonders bevorzugte erfindungsgemäße Mittel enthalten
- 0,1 bis 15 Gew.-% Behenoyl PG Trimonium Chloride und
- 0,1 bis 15 Gew.-% Behentrimonium Chloride und
- 0,1 bis 15 Gew.-% Quaternium-87 und
- 0,1 bis 15 Gew.-% Dimethylacrylamide/Ethyltrimonium Chloride Methacrylate Copolymer und
- 0,5 bis 2,5 Gew.-% Isopropylmyristat.

Ganz besonders bevorzugte Mittel enthalten sowohl Spreitmittel als auch Fettstoff(e), vorzugsweise Ester von Octansäure mit langkettigen Fettalkoholen.

Ganz besonders bevorzugte erfindungsgemäße Mittel enthalten
- 0,1 bis 15 Gew.-% Behenoyl PG Trimonium Chloride und
- 0,1 bis 15 Gew.-% Behentrimonium Chloride und
- 0,1 bis 15 Gew.-% Quaternium-87 und
- 0,1 bis 15 Gew.-% Dimethylacrylamide/Ethyltrimonium Chloride Methacrylate Copolymer und
- 0,1 bis 10 Gew.-% Ester von Kokosfettalkohlen mit Octansäure und
- 0,5 bis 2,5 Gew.-% Isopropylmyristat.

Die erfindungsgemäßen Mittel können weitere Inhaltsstoffe enthalten.

Mit besonderem Vorzug enthalten die erfindungsgemäßen Mittel amphotere(s) Tensid(e). Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Bevorzugte erfindungsgemäße Haarbehandlungsmittel sind dadurch gekennzeichnet, dass sie 1 bis 30 Gew.-%, vorzugsweise 6 bis 25 Gew.-%, weiter bevorzugt 7 bis 20 Gew.-%, noch weiter bevorzugt 8 bis 15 Gew.-% und insbesondere 10 bis 12,5 Gew.-% amphotere(s) Tensid(e) enthalten.

Besonders bevorzugte erfindungsgemäße Haarbehandlungsmittel sind dadurch gekennzeichnet, dass sie amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ - Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCl-Bezeichnung Cocamidopropyl Betain bekannten Verbindungen,
- der unter der INCI-Bezeichnung Disodium Cocoamphodiacetate bekannten Verbindungen
enthalten, wobei bevorzugte Mittel das bzw. die amphotere(n) Tensid(e) in Mengen von 0,5 bis 9 Gew.-%, vorzugsweise von 0,75 bis 8 Gew.-% und insbesondere von 1 bis 7,5 Gew.-%, jeweils bezogen auf das gesamte Mittel, enthalten.

Besonders bevorzugte Haarbehandlungsmittel enthalten als amphotere Tenside Betaine der Formel (Bet-I) 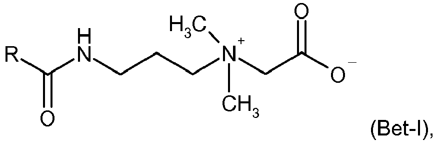 in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCl-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:

H₃C-(CH₂)₇-C(O)-NH-(CH₂)₃N⁺(CH₃)₂CH₂COO⁻

H₃C-(CH₂)₉-C(O)-NH-(CH₂)₃N⁺(CH₃)₂CH₂COO⁻

H₃C-(CH₂)₁₁-C(O)-NH-(CH₂)₃N⁺(CH₃)₂CH₂COO⁻

H₃C-(CH₂)₁₃-C(O)-NH-(CH₂)₃N⁺(CH₃)₂CH₂COO⁻

H₃C-(CH₂)₁₅-C(O)-NH-(CH₂)₃N⁺(CH₃)₂CH₂COO⁻

H₃C-(CH₂)₇-CH=CH-(CH₂)₇-C(O)-NH-(CH₂)₃N⁺(CH₃)₂CH₂COO⁻

Besonders bevorzugt werden Tenside der Formel (Bet-I) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (Bet-I) enthalten.

Zusätzlich zu dem bzw. den Amphotensiden der Formel (Bet-I) oder an deren Stelle können die erfindungsgemäßen Haarbehandlungsmittel mit besonderem Vorzug als amphotere Tenside Betaine der Formel (Bet-II) 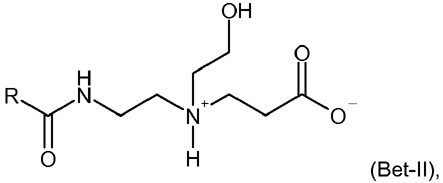 enthalten, in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amphoacetate bezeichnet, wobei die Vertreter, die sich von Kokosfettsäuren ableiten bevorzugt sind und als Cocoamphoactetate bezeichnet werden.

Aus herstellungstechnischen Gründen enthalten Tenside dieses Typs immer auch Betaine der Formel (Bet-IIa)

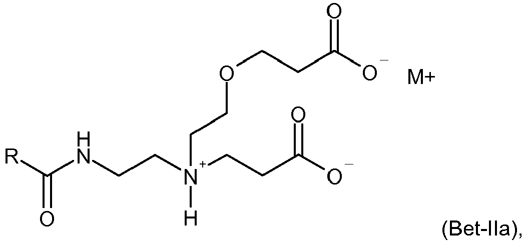

in der R für einen geradkettigen oder verzweigten, gesättigten oder ein- bzw. mehrfach ungesättigten Alkyl- oder Alkenlyrest mit 8 bis 24 Kohlenstoffatomen und M für ein Kation steht. Diese Tenside werden nach der INCI-Nomenklatur als Amphodiacetate bezeichnet, wobei die Vertreter, die sich von Kokosfettsäuren ableiten, bevorzugt sind und als Cocoamphodiactetate bezeichnet werden.

Besonders bevorzugt werden erfindungsgemäß Tenside der Formel (Bet-II) eingesetzt, die ein Gemisch der folgenden Vertreter sind:

H₃C-(CH₂)₇-C(O)-NH-(CH₂)₂NH⁺(CH₂CH₂OH)CH₂CH₂COO⁻

H₃C-(CH₂)₉-C(O)-NH-(CH₂)₂NH⁺(CH₂CH₂OH)CH₂CH₂COO⁻

H₃C-(CH₂)₁₁-C(O)-NH-(CH₂)₂NH⁺(CH₂CH₂OH)CH₂CH₂COO⁻

H₃C-(CH₂)₁₃-C(O)-NH-(CH₂)₂NH⁺(CH₂CH₂OH)CH₂CH₂COO⁻

H₃C-(CH₂)₁₅-C(O)-NH-(CH₂)₂NH⁺(CH₂CH₂OH)CH₂CH₂COO⁻

H₃C-(CH₂)₇-CH=CH-(CH₂)₇-C(O)-NH-(CH₂)₂NH⁺(CH₂CH₂OH)CH₂CH₂COO⁻

Besonders bevorzugt werden Tenside der Formel (Bet-II) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (Bet-II) enthalten.

Zusätzlich zu den amphoteren Tensiden oder an ihrer Stelle können die erfindungsgemäßen Mittel auch nichtionische Tenside und/oder kationische Tenside (siehe oben) enthalten.

Besonders bevorzugte nichtionische Tenside sind Alkylpolyglycoside. Alkylpolyglycoside (APG) sind nichtionische Tenside, die vollständig aus nachwachsenden Rohstoffen (Zuckerbausteine, vorwiegend Glucose z.B. aus Maisstärke und Fettalkohol z.B. aus Kokosöl) hergestellt werden. Alkylpolyglycoside sind durch sauer katalysierte Reaktion (Fischer-Reaktion) von Zuckern, insbesondere Glucose (oder Stärke) oder von Butylglycosiden mit Fettalkoholen zugänglich.

Dabei entstehen komplexe Gemische aus Alkylmonoglucosid (Alkyl-α-D- und -β-D-glucopyranosid sowie geringe Anteile -glucofuranosid), Alkyldiglucosiden (-isomaltoside, -maltoside etc.) und Alkyloligoglucosiden (-maltotrioside, -tetraoside etc.). Der durchschnittliche Polymerisationsgrad kommerzieller Produkte, deren Alkyl-Reste im Bereich C8-C16 liegen, beträgt 1,2-1,5.

Erfindungsgemäß bevorzugt werden Alkylpolyglycoside entsprechend der allgemeinen Formel RO-(Z)ₓ eingesetzt, wobei R für Alkyl, Z für Zucker sowie x für die Anzahl der Zuckereinheiten steht. Besonders bevorzugt sind solche Alkylpolyglycoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen
besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose. Demnach sind bevorzugte erfindungsgemäße Haarbehandlungsmittel dadurch gekennzeichnet, dass sie- bezogen auf ihr Gewicht - 0,1 bis 20 Gew.-%, vorzugsweise 1 bis 10 Gew.-% und insbesondere 2 bis 8 Gew.-% Alkylpolyglycosid(e) der Formel

**H₃C⁻(CHz)ₙ-O-(Z)ₓ**

enthalten, in der n für Werte von 5 bis 21, vorzugsweise von 7 bis 19, besonders bevorzugt von 9 bis 17 und insbesondere von 11 bis 13 und k für Werte von 1,1 bis 1,8, vorzugsweise von 1,2 bis 1,5 stehen, und Z für einen Zuckerbaustein aus der Gruppe Glucose, Fructose, Galactose, Arabinose, Ribose, Xylose, Lyxose, Allose, Altrose, Mannose, Gulose, Idose, Talose und Sucrose steht.

Glucose ist ein besonders bevorzugter Zuckerbaustein (Z), so dass bevorzugte erfindungsgemäße Haarbehandlungsmittel dadurch gekennzeichnet sind, dass sie- bezogen auf ihr Gewicht - 0,1 bis 15 Gew.-%, vorzugsweise 1 bis 10 Gew.-% und insbesondere 2 bis 8 Gew.-% Alkylpolyglucosid(e) der Formel 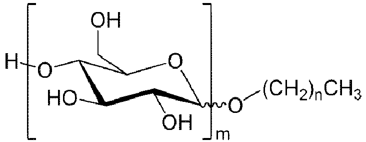 enthalten, in der n für Werte von 5 bis 21, vorzugsweise von 7 bis 19, besonders bevorzugt von 9 bis 17 und insbesondere von 11 bis 13 und m für Werte von 1,1 bis 1,8, vorzugsweise von 1,2 bis 1,5 stehen.

Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.

Ganz besonders bevorzugte Alkypolyglucoside sind solche, deren Alkylrest ein Laurylrest ist. Bei Substanzgemischen aus nativen Quellen sind solche Quellen bevorzugt, die einen hohen Anteil an C12-Fettsäuren haben, insbesondere Cocosfettsäuren. Demnach sind besonders bevorzugte erfindungsgemäße Haarbehandlungsmittel dadurch gekennzeichnet, dass sie- bezogen auf ihr Gewicht - 0,1 bis 15 Gew.-%, vorzugsweise 1 bis 10 Gew.-% und insbesondere 2 bis 8 Gew.-% Alkylpolyglucosid(e) enthalten, in der n für den Wert 11 m für Werte von 1,1 bis 1,8, vorzugsweise von 1,2 bis 1,5 stehen.

Die Pflegeffekte der erfindungsgemäßen Mittel lassen sich noch weiter verstärken, indem bestimmte Pflegestoffe eingesetzt werden. Vorzugsweise werden diese aus bestimmten Gruppen an sich bekannter Pflegestoffe ausgewählt, da diese Pflegestoffe formulierungstechnisch und vom Pflegeffekt hervorragend mit der erfindungsgemäßen Kombination harmonieren. Erfindungsgemäß bevorzugte Haarbehandlungsmittel sind dadurch gekennzeichnet, dass sie zusätzlich Pflegestoff(e) - bezogen auf ihr Gewicht - in Mengen von 0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% enthalten, wobei bevorzugte Pflegstoff(e) ausgewählt sind aus der Gruppe
i.L-Carnitin und/oder seiner Salze;
ii.Taurin und/oder seiner Salze;
iii.Niacinamid;
iv.Ubichinon
v.Ectoin;

L-Carnitin (IUPAC-Name(*R*)-(3-Carboxy-2-hydroxypropyl)- *N*,*N*,*N*-trimethylammoniumhydroxid), ist eine natürlich vorkommende, vitaminähnliche Substanz.

Als Betain kann L-Carnitin Additionsverbindungen und Doppelsalze bilden. Erfindungsgemäß bevorzugte L-Carnitinderivate sind insbesondere ausgewählt aus Acetyl-L-Carnitin, L-Carnitin-Fumarat, L-Carnitin-Citrat, Lauroyl-L-Carnitin und besonderes bevorzugt L-Carnitin-Tartrat. Die genannten L-Carnitin-Verbindungen sind beispielsweise von der Firma Lonza GmbH (Wuppertal, Deutschland) erhältlich.

Erfindungsgemäße bevorzugte Haarbehandlungsmittel sind dadurch gekennzeichnet, dass sie - bezogen auf ihr Gewicht -0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.

Ein weiterer, bevorzugter einsetzbarer Pflegestoff, der aktivierende Eigenschaften besitzt, ist das Taurin. Erfindungsgemäß bevorzugte Haarbehandlungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere bevorzugte Gruppe von Pflegestoffen in den erfindungsgemäßen Mitteln sind Vitamine, Provitamine oder Vitaminvorstufen. Diese werden nachfolgend beschrieben:
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester,
Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt (siehe weiter unten). Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße Haarbehandlungsmittel bevorzugt, die - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel -2,4-Dihydroxy-*N*-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es hat sich gezeigt, dass bestimmte Chinone eine besondere Eignung als Pflegestoff besitzen. Als weiteren Pflegestoff können die erfindungsgemäßen Mittel daher 0,0001 bis 5 Gew.-% mindestens eines Biochinons der Formel (Ubi) 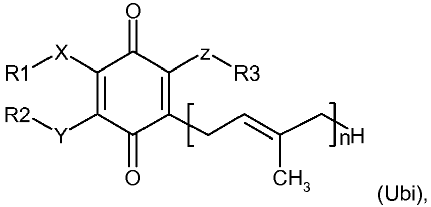 enthalten in der
X, Y, Z stehen unabhängig voneinander für -O- oder -NH- oder NR⁴- oder eine chemische Bindung
R¹, R², R³ stehen unabhängig voneinander für ein Wasserstoffatom oder eine gegebenenfalls substituierte Arylgruppe oder eine gegebenenfalls substituierte (C₁-C₆)-Alkylgruppe oder eine Hydroxyalkylgruppe oder eine Polyhydroxyalkylgruppe oder eine gegebenenfalls substituierte (C₁-C₆)-Alkylengruppe, oder einen (C₁-C₆)-Acylrest, wobei bevorzugte Reste unabhängig voneinander ausgewählt sind aus -H, -CH₃, -CH₂CH₃, -(CH₂)₂CH₂, -CH(CH₃)₂,-(CH₂)₃CH₃, -CH(CH₃)CH₂CH₃, -CH₂CH(CH₃)₂, -C(CH₃)₃
R⁴ steht für -CH₃, -CH₂CH₃, -(CH₂)₂CH₂, -CH(CH₃)₂,-(CH₂)₃CH₃, -CH(CH₃)CH₂CH₃, -CH₂CH(CH₃)₂, -C(CH₃)₃
n steht für Werte von 1 bis 20, vorzugsweise von 2 bis 15 und insbesondere für 5, 6, 7, 8, 9, 10.

Besonders bevorzugte erfindungsgemäße Haarbehandlungsmittel sind dadurch gekennzeichnet, dass sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 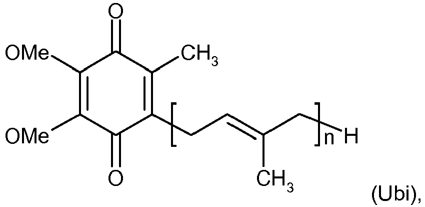 in der n für die Werte = 6, 7, 8, 9 oder 10, besonders bevorzugt für 10 (Coenzym Q10) steht.

Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel dadurch gekennzeichnet, dass sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons der Formel Ubi-b enthalten

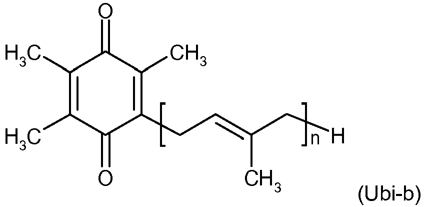

in der n für Werte von 1 bis 20, vorzugsweise von 2 bis 15 und insbesondere für 5, 6, 7, 8, 9, 10 steht, wobei besonders bevorzugt Mittel Plastochinon PQ-9 enthalten.

Als weiteren Pflege-Enhancer können die erfindungsgemäßen Mittel Ectoin enthalten. Ectoin ((4S)-2-Methyl-1,4,5,6-Tetrahydropyrimidin-4-Carbonsäure) ist ein zur Gruppe der kompatiblen Solute gehörender Naturstoff. Die stark wasserbindende niedermolekulare organische Verbindung tritt in halophilen Bakterien auf und ermöglicht diesen extremophilen Organismen unter Stressbedingungen zu überleben. Erfindungsgemäß bevorzugte Haarbehandlungsmittel sind dadurch gekennzeichnet, dass sie - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.

Zur Verbesserung der Elastizität und Festigung der inneren Struktur der mit erfindungsgemäßen Mitteln behandelter Haare können die erfindungsgemäßen Mittel Purin und/oder Purinderivate als Pflegestoff enthalten. Insbesondere die Kombination von Purin und/oder Purinderivaten mit Ubichinonen und/oder Plastochinonen als Pflegestoff führt dazu, dass die mit entsprechenden Mitteln behandelten Haare unter anderem höhere Meßwerte bei der Differenzthermoanalyse und verbesserte Nass- und Trockenkämmbarkeiten zeigen.

Purin (7*H*-Imidazo[4,5-*d*]pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten. Purin kann beispielsweise aus Aminoacetonitril und Formamid hergestellt werden. Purine und Purinderivate werden oft aus Naturstoffen isoliert, sind aber auch auf vielen Wegen synthetisch zugänglich.

Bevorzugte erfindungsgemäße Mittel enthalten Purin und/oder Purinderivate in engeren Mengenbereichen. Hier sind erfindungsgemäß bevorzugte kosmetische Mittel dadurch gekennzeichnet, dass sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.

Unter Purin, den Purinen und den Purinderivaten sind erfindungsgemäß einige Vertreter besonders bevorzugt. Erfindungsgemäß bevorzugte Haarbehandlungsmittel sind dadurch gekennzeichnet, dass sie als Pflegestoff - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthält, wobei bevorzugte Mittel Purin und/oder Purinderivat(e) der Formel (Pur-I) enthalten 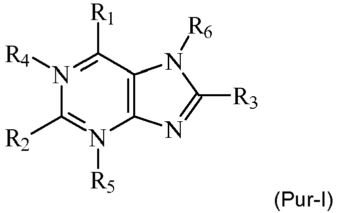 in der die Reste R¹, R² und R³ unabhängig voneinander ausgewählt sind aus -H, - OH, NH₂, -SH und die Reste R⁴, R⁵ und R⁶ unabhängig voneinander ausgewählt sind aus -H, -CH₃ und -CH₂-CH₃, wobei folgende Verbindungen bevorzugt sind:
Purin (R¹ = R² = R³ = R⁴ = R⁵ = R⁶ = H), Adenin (R¹ = NH₂, R² = R³ = R⁴ = R⁵ = R⁶ = H), Guanin (R¹ = OH, R² = NH₂, R³ = R⁴ = R⁵ = R⁶ = H), Harnsäure (R¹ = R² = R³ = OH, R⁴ = R⁵ = R⁶ = H), Hypoxanthin (R¹ = OH, R² = R³ = R⁴ = R⁵ = R⁶ = H), 6-Purinthiol (R¹ = SH, R² = R³ = R⁴ = R⁵ = R⁶ = H), 6-Thioguanin (R¹ = SH, R² = NH₂, R³ = R⁴ = R⁵ = R⁶ = H), Xanthin (R¹ = R² = OH, R³ = R⁴ = R⁵ = R⁶ = H), Coffein (R¹ = R² = OH, R³ = H, R⁴ = R⁵ = R⁶ = CH₃), Theobromin (R¹ = R² = OH, R³ = R⁴ = H, R⁵ = R⁶ = CH₃), Theophyllin (R¹ = R² = OH, R³ = H, R⁴ = CH₃, R⁵ = CH₃, R⁶ = H).

Es ist weiterhin vorteilhaft, Purin bzw. Purinderivate und Biochinone in einem bestimmten Verhältnis zueinander einzusetzen. Hier sind erfindungsgemäße Mittel bevorzugt, bei denen das Gewichtsverhältnis von Purin(derivat(en)) und Biochinon(en) 10:1 bis 1:100, vorzugsweise 5:1 bis 1:50, besonders bevorzugt 2:1 bis 1:20 und insbesondere 1:1 bis 1:10 beträgt.

Wie bereits erwähnt, ist Coffein ein besonders bevorzugtes Purinderivat, und das Coenzym Q10 ist ein besonders bevorzugtes Biochinon. Besonders bevorzugte erfindungsgemäße Mittel sind daher dadurch gekennzeichnet, dass sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Coffein und 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% Coenzym Q10 enthalten.

Als Pflegestoff können die erfindungsgemäßen Mittel auch Flavonoide enthalten. Die Flavonoide sind eine Gruppe von wasserlöslichen Pflanzenfarbstoffen und spielen eine wichtige Rolle im Stoffwechsel vieler Pflanzen. Sie gehören zusammen mit den Phenolsäuren zu den Polyphenolen. Es sind weit über 6500 unterschiedliche Flavonoide bekannt, die sich in Flavonole, Flavone, Flavanone, Isoflavonoide und Anthocyane einteilen lassen.

Erfindungsgemäß können Flavonoide aus allen sechs Gruppen eingesetzt werden, wobei bestimmte Vertreter aus den einzelnen Gruppen als Pflegestoff wegen ihrer besonders intensiven Wirkung bevorzugt sind. Bevorzugte Flavonole sind Quercetin, Rutin, Kaempferol, Myricetin, Isorhamnetin, bevorzugte Flavanole sind Catechin, Gallocatechin, Epicatechin, Epigallocatechingallat , Theaflavin, Thearubigin, bevorzugte Flavone sind Luteolin, Apigenin, Morin, bevorzugte Flavanone sind Hesperetin, Naringenin, Eriodictyol, bevorzugte Isoflavonoide sind Genistein, Daidzein, und bevorzugte Anthocyanidine (Anthocyane) sind Cyanidin, Delphinidin, Malvidin, Pelargonidin, Peonidin, Petunidin.

Erfindungsgemäß besonders bevorzugte Haarbehandlungsmittel sind dadurch gekennzeichnet, dass sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Flavonoide, insbesondere Flavonole, besonders bevorzugt 3,3',4',5,7-Pentahydroxyflavon (Quercetin) und/oder 3,3',4',5,7-Pentahydroxyflavon-3-*O*-rutinosid (Rutin), enthalten.

Bevorzugt ist auch der Einsatz von Bisabolol und/oder Bisabololoxiden als Pflegestoff in den erfindungsgemäßen Mitteln. Hier sind erfindungsgemäße Haarbehandlungsmittel bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol 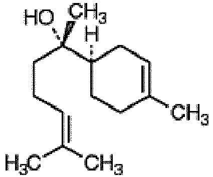 enthalten.

Auch Creatin eignet sich erfindungsgemäß als Pflegestoff. Creatin (3-Methylguanidinoessigsäure) ist eine organische Säure, die in Wirbeltieren u. a. zur Versorgung der Muskeln mit Energie beiträgt. Kreatin wird in der Niere, der Leber und in der Bauchspeicheldrüse synthetisiert. Sie leitet sich formal von den Aminosäuren Glycin und Arginin ab und ist zu 95 % im Skelettmuskel vorhanden. Besonders bevorzugte erfindungsgemäße Haarbehandlungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% *N*-Methylguanidino-essigsäure (Creatin).

Die erfindungsgemäßen Mittel können zusätzlich zu den vorstehend genannten Inhaltsstoffen und optionalen weiteren Inhaltsstoffen weitere Stoffe enthalten, die Haarausfall verhindern, lindern oder heilen. Insbesondere ist ein Gehalt an haarwurzelstabilisierenden Wirkstoffen vorteilhaft. Diese Stoffe werden nachstehend beschrieben:
Propecia (Finasterid) ist das zur Zeit einzige Präparat, das weltweit zugelassen ist und für das in zahlreichen Studien eine Wirksamkeit und Verträglichkeit nachgewiesen wurde. Propecia bewirkt, dass sich weniger DHT aus Testosteron bilden kann.

Minoxidil ist mit oder ohne ergänzende Zusatzstoffe das wohl älteste nachweislich wirkende Haarwuchsmittel. Zur Behandlung von Haarausfall darf es nur zur äußeren Anwendung verwendet werden. Es gibt Haarwasser, die 2%-5% Minoxidil enthalten, außerdem Gels mit bis zu 15% Minoxidil. Die Wirksamkeit nimmt mit der Dosierung zu, in Haarwassern ist Minoxidil jedoch nur bis zu 5% Anteil löslich. In vielen Ländern sind Haarwasser mit bis zu 2% Minoxidilgehalt verschreibungsfrei erhältlich.

Zur Bekämpfung der hormonellen Einflüße auf die Haarfollikel kann zur äußeren Anwendung Spironolactone in Form von Haarwasser und in Kombination mit Minoxidil angewandt werden. Spironolactone wirkt als Androgen-Rezeptor-Blocker, d. h. die Bindung von DHT an die Haarfollikel wird verhindert.

Zusammenfassend sind erfindungsgemäße Haarbehandlungsmittel bevorzugt, die zusätzlich - bezogen auf sein Gewicht - 0,001 bis 5 Gew.-% Haarwurzel-stabilisierende Stoffe, insbesondere Minoxidil und/oder Finasterid und/oder Ketoconazol enthalten.

Zusätzlich zu den Pflegestoffen können di erfindungsgemäßen Mittel weitere Pflegestoffe enthalten. Deren Anwesenheit ist für die Erzielung der erfindungsgemäßen Effekte nicht zwingend erforderlich, doch können weitergehende Effekte, wie ein angenehmer Griff oder eine angenehme Applikationshaptik aus dem Einsatz dieser Pflegestoffe resultieren.

Als weiteren Inhaltsstoff können die erfindungsgemäßen Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte Haarbehandlungsmittel dadurch gekennzeichnet, dass sie als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanain und/oder Valin und/oder Lysin und/oder Leucin und/oder Threonin enthalten.

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Haarbehandlungsmittel dadurch gekennzeichnet, dass sie als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus
- Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose
- Disacchariden, insbesondere Saccharose und/oder Maltose und/oder Lactose und/oder Trehalose und/oder Cellobiose und/oder Gentiobiose und/oder Isomaltose.

Wie bereits erwähnt, enthalten bevorzugte erfindungsgemäße Mittel (eine) Aminosäure(n).

Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β -Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte kosmetische Mittel dadurch gekennzeichnet, dass sie zusätzlich - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthalten.

Eine besonders bevorzugte Gruppe von Inhaltsstoffen stellen die Silikone dar.

Erfindungsgemäße bevorzugte Mittel sind dadurch gekennzeichnet, dass sie mindestens ein Silicon, vorzugsweise ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon
enthält;
oder deren Gemischen.

Erfindungsgemäß besonders bevorzugte Mittel enthalten das bzw. die Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.

Bevorzugte Silikone werden nachstehend beschrieben.

Besonders bevorzugte erfindungsgemäße Mittel sind dadurch gekennzeichnet, dass sie mindestens ein Silikon der Formel Si-I

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht.

Diese Silikone werden nach der INCI-Nomenklatur als DIMETHICONE bezeichnet. Es werden im Rahmen der vorliegenden Erfindung als Silicon der Formel Si-I die Verbindungen (CH₃)₃Si--O-Si(CH₃)₃, (CH₃)₃Si-O-(CH₃)₂Si-O-Si(CH₃)₃ und/oder (CH₃)₃Si-[O-(CH₃)₂Si]₂-OO-Si(CH₃)₃ besonders bevorzugt eingesetzt.

Selbstverständlich können auch Mischungen der o.g. Silikone in den erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind.

Ganz besonders bevorzugte erfindungsgemäße Haarbehandlungsmittel sind dadurch gekennzeichnet, dass sie - bezogen auf ihr Gewicht - 0,1 bis 10 Gew.-%, vorzugsweise 0,15 bis 7,5 Gew.-%, weiter bevorzugt 0,2 bis 5 Gew.-%, noch weiter bevorzugt 0,25 bis 2,5 Gew.-% und insbesondere 0,4 bis 1,5 Gew.-% Polydimethysiloxan (INCI-Bezeichnung: Dimethicone) enthalten. Insbesondere bevorzugte erfindungsgemäße Haarbehandlungsmittel sind dadurch gekennzeichnet, dass sie bezogen auf ihr Gewicht
a) 0,5 bis 2,5 Gew.-% Behenoyl PG Trimonium Chloride,
b) 0,5 bis 2,5 Gew.-% Behentrimonium Chloride,
c) 0,5 bis 2,5 Gew.-% Quaternium-87,
d) 0,5 bis 2,5 Gew.-% Dimethylacrylamide/Ethyltrimonium Chloride Methacrylate Copolymer,
e) 0,5 bis 2,5 Gew.-% Isopropylmyristat
f) 0,4 bis 1,5 Gew.-% Dimethicone
enthalten.

Besonders bevorzugte erfindungsgemäße Mittel enthalten ein oder mehrere aminofunktionelle Silicone. Solche Silicone können z.B. durch die Formel

M(RₐQ_{b}SiO_{(4-a-b)/2)x}(R_{c}SiO_{(4-c)/2)y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen,-CH₂CH(CH₃)CH₂-, Phenylen, Naphthylen, -CH₂CH₂SCH₂CH₂-, -CH₂CH₂OCH₂-, -OCH₂CH₂-,-OCH₂ CH₂CH₂-, -CH₂CH(CH₃)C(O)OCH₂-, -(CH₂)₃CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfasst, wie Piperazinyl. Z ist am bevorzugtesten ein -NHCH₂CH₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b}SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Besonders bevorzugte erfindungsgemäße Mittel sind dadurch gekennzeichnet, dass sie mindestens ein aminofunktionelles Silikon der Formel (Si-IIa) 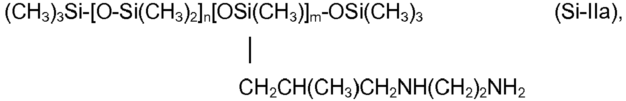 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet. Besonders bevorzugt sind auch erfindungsgemäße Mittel, die ein aminofunktionelles Silikon der Formel (Si-IIb) 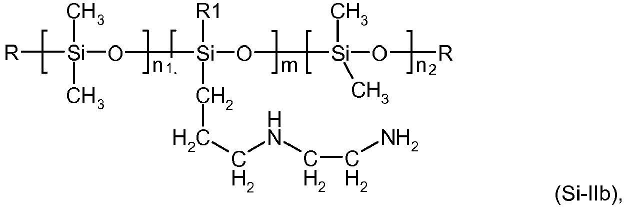 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons.

Sie kann durch Titration ermittelt und auch in der Einheit mg KOH/g angegeben werden.
Erfindungsgemäß bevorzugte Mittel sind dadurch gekennzeichnet, dass sie, bezogen auf ihr Gewicht, 0,01 bis 10 Gew.-%, vorzugsweise 0,1 bis 8 Gew.-%, besonders bevorzugt 0,25 bis 7,5 Gew.-% und insbesondere 0,5 bis 5 Gew.-% aminofunktionelle(s) Silikon(e) enthalten.

Auch die nach INCI als CYCLOMETHICONE bezeichneten cyclischen Dimethicone sind erfindungsgemäß mit Vorzug einsetzbar. Hier sind erfindungsgemäße Mittel bevorzugt, die mindestens ein Silikon der Formel Si-III

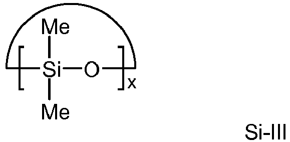

enthalten, in der x für eine Zahl von 3 bis 200, vorzugsweise von 3 bis 10, weiter bevorzugt von30 bis 7 und insbesondere 3, 4, 5 oder 6, steht.

Die vorstehend beschriebenen Silicone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Erfindungsgemäß ebenfalls bevorzugte Mittel sind dadurch gekennzeichnet, dass sie mindestens ein Silikon der Formel Si-IV

R₃Si-[O-SiR₂]ₓ-(CH₂)ₙ-[O-SiR₂]_{y}-O-SiR₃ (Si-IV),

enthalten, in der R für gleiche oder verschiedene Reste aus der Gruppe -H,-Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugte Mittel sind dadurch gekennzeichnet, dass sie mindestens ein wasserlösliches Silikon enthalten.

Aus ästhetischen Gründen werden "klare" Produkte von Verbrauchern oft bevorzugt. Erfindungsgemäß bevorzugte Haarbehandlungsmittel sind daher dadurch gekennzeichnet, dass sie transparent bzw. transluzent sind.

Unter transparent oder transluzent wird im Rahmen der vorliegenden Erfindung eine Zusammensetzung verstanden, die einen NTU-Wert von unter 100 aufweist. Der NTU-Wert (Nephelometric Turbidity Unit, *Nephelometrischer Trübungswert*; NTU) ist eine in der Wasseraufbereitung verwendete Einheit für Trübungsmessungen in Flüssigkeiten. Sie ist die Einheit einer mit einem kalibriertem Nephelometer gemessenen Trübung einer Flüssigkeit.

Weiterhin kann in einer bevorzugten Ausführungsform der Erfindung ein erfindungsgemäßes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul®M 40, Uvasorb®MET, Neo Heliopan®BB, Eusolex®4360), 2-Phenylbenzimidazol-5-sulfonsäure und deren Kalium-, Natrium- und Triethanolaminsalze (Phenylbenzimidazole sulfonic acid; Parsol®HS; Neo Heliopan®Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol®1789, Eusolex®9020), α-(2-Oxoborn-3-yliden)-toluol-4-sulfonsäure und deren Salze, ethoxylierte 4-Aminobenzoesäure-ethylester (PEG-25 PABA; Uvinul®P 25), 4-Dimethylaminobenzoesäure-2-ethylhexylester (Octyl Dimethyl PABA; Uvasorb®DMO, Escalol®507, Eusolex®6007), Salicylsäure-2-ethylhexylester (Octyl Salicylat; Escalol®587, Neo Heliopan®OS, Uvinul®018), 4-Methoxyzimtsäure-isopentylester (Isoamyl p-Methoxycinnamate; Neo Heliopan®E 1000), 4-Methoxyzimtsäure-2-ethylhexyl-ester (Octyl Methoxycinnamate; Parsol®MCX, Escalol®557, Neo Heliopan®AV), 2-Hydroxy-4-methoxybenzophenon-5-sulfonsäure und deren Natriumsalz (Benzophenone-4; Uvinul®MS 40; Uvasorb®S 5), 3-(4'-Methylbenzyliden)-D,L-Campher (4-Methylbenzylidene camphor; Parsol®5000, Eusolex®6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb®20 H, Uvinul®400), 1,1'-Diphenylacrylonitrilsäure-2-ethylhexyl-ester (Octocrylene; Eusolex®OCR, Neo Heliopan®Type 303, Uvinul®N 539 SG), o-Aminobenzoesäure-menthylester (Menthyl Anthranilate; Neo Heliopan®MA), 2,2',4,4'-Tetrahydroxybenzophenon (Benzophenone-2; Uvinul®D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV-Filters durch eine kationische Gruppe Q, insbesondere mit einer quartären Aminofunktion, ersetzt wird.

Verbindungen, von denen sich der Strukturteil U ableiten kann, sind beispielsweise substituierte Benzophenone, p-Aminobenzoesäureester, Diphenylacrylsäureester, Zimtsäureester, Salicylsäureester, Benzimidazole und o-Aminobenzoesäureester.

Strukturteile U, die sich vom Zimtsäureamid oder vom N,N-Dimethylamino-benzoesäureamid ableiten, sind erfindungsgemäß bevorzugt.

Die Strukturteile U können prinzipiell so gewählt werden, daß das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, dass der molare Extinktionskoeffizient des UV-Filters am Absorptionsmaximum oberhalb von 15 000, insbesondere oberhalb von 20000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so dass der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur -(CH₂)ₓ-N⁺R¹R²R³ X⁻, in der x steht für eine ganze Zahl von 1 bis 4, R¹ und R² unabhängig voneinander stehen für C₁₋₄-Alkylgruppen, R³ steht für eine C₁₋₂₂-Alkylgruppe oder eine Benzylgruppe und X⁻ für ein physiologisch verträgliches Anion. Im Rahmen dieser allgemeinen Struktur steht x bevorzugt für die die Zahl 3, R¹ und R² jeweils für eine Methylgruppe und R³ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyl-trimethylammoniumchlorid (Incroquat®UV-283) und Dodecyldimethylaminobenzamidopropyl-dimethylammoniumtosylat (Escalol® HP 610).

Selbstverständlich umfasst die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter (I) sind in den erfindungsgemäßen Mitteln üblicherweise in Mengen 0,1-5 Gew.-%, bezogen auf das gesamte Mittel, enthalten. Mengen von 0,4-2,5 Gew.-% sind bevorzugt.

Die erfindungsgemäßen Mittel können weiterhin eine 2-Pyrrolidinon-5-carbonsäure und deren Derivate (J) enthalten. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf das gesamte Mittel, besonders bevorzugt 0,1 bis 5, und insbesondere 0,1 bis 3 Gew.-%.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Vorteilhaft im Sinne der Erfindung können zusätzlich kurzkettige Carbonsäuren (N) den Wirkstoffkomplex (A) unterstützen. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäß verwendeten Carbonsäuren können selbstverständlich entlang der Kohlenstoffkette oder des Ringgerüstes substituiert sein. Zu den Substituenten der erfindungsgemäß verwendeten Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-,C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in α-Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Als weiterhin vorteilhaft hat es sich gezeigt, wenn die erfindungsgemäßen Mittel weitere Polymere, vorzugsweise anionische und/oder nichtionische Polymere enthalten.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik®11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen. Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.

Die erfindungsgemäßen Mittel können in einer weiteren Ausführungsform nichtionogene Polymere (G4) enthalten.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) und Natrosol®-Typen (Hercules) vertrieben werden.
- Stärke und deren Derivate, insbesondere Stärkeether, beispielsweise Structure® XL (National Starch), eine multifunktionelle, salztolerante Stärke;
- Schellack
- Polyvinylpyrrolidone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone.

Es ist erfindungsgemäß auch möglich, dass die Zubereitungen mehrere, insbesondere zwei verschiedene Polymere gleicher Ladung und/oder jeweils ein ionisches und ein amphoteres und/oder nicht ionisches Polymer enthalten.

Die weiteren Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Bezüglich weiterer fakultativer Komponenten sowie die eingesetzten Mengen dieser Komponenten wird ausdrücklich auf die dem Fachmann bekannten einschlägigen Handbücher verwiesen.

Wie bereits erwähnt, kommt der hohen Pflegewirkung der erfindungsgemäßen Mittel insbesondere daher Bedeutung zu, als sie auch in Gegenwart von Oxidationsmitteln - beispielsweise im Rahmen der oxidativen Haarfärbung - hervorragende Ergebnisse liefert. Ebenfalls offenbart ist ein Verfahren zur Behandlung von keratinischen Fasern, insbesondere menschlichen Haaren, bei dem ein erfindungsgemäßes Haarbehandlungsmittel auf keratinische Fasern aufgebracht und dort entweder bis zur nächsten Haarwäsche belassen (sogenanntes "leave-on"-Produkt) oder nach einer Einwirkzeit von 30 bis 300 Sekunden ausgespült (sogenanntes "rinse-off"-Produkt) wird.

## Patentansprüche

1. Haarbehandlungsmittel, enthaltend
a) mindestens eine quartäre Ammoniumverbindung (QAV) gemäß der Formel I 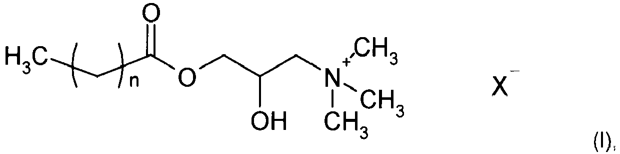 in der n für eine ganze Zahl von 10 bis 24 und X⁻ für ein Anion steht,
b) mindestens eine quartäre Ammoniumverbindung (QAV) gemäß der Formel II 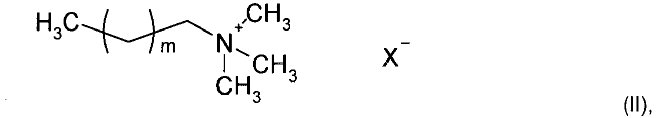 in der m für eine ganze Zahl von 10 bis 24 und X⁻ für ein Anion steht,
c) mindestens eine quartäre Ammoniumverbindung (QAV) der Formel III 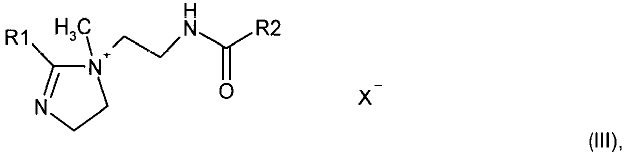 in der R1 und R2 unabhängig voneinander jeweils für einen gesättigten oder ungesättigten, linearen oder verzweigten Kohlenwasserstoffrest mit einer Kettenlänge von 16 bis 30 Kohlenstoffatomen stehen und X⁻ für ein Anion steht,
d) mindestens ein kationisches Copolymer, enthaltend Monomere der Formel (IV) und Monomere der Formel (V) 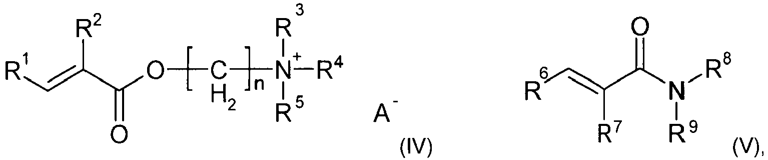 in denen R¹ bis R⁹ unabhängig voneinander Wasserstoff, C₁₋₄-Alkyl wie Methyl, Ethyl, Propyl, iso-Propyl, Butyl, iso-Butyl oder tert-Butyl bedeuten mit der Maßgabe, dass mindestens einer der Reste R⁶, R⁷, R⁸ oder R⁹ C₁₋₄-Alkyl bedeutet, n für ganze Zahlen von 1 bis 8, und A⁻ für ein physiologisch verträgliches Anion steht.

2. Haarbehandlungsmittel nach Anspruch 1, **dadurch gekennzeichnet, dass** es - bezogen auf sein Gewicht - zusätzlich 0,1 bis 20 Gew.-%, vorzugsweise 0,2 bis 10 Gew.-%, weiter bevorzugt 0,3 bis 7,5 Gew.-%, noch weiter bevorzugt 0,4 bis 5 Gew.-% und insbesondere 0,5 bis 2,5 Gew.-% Isopropylmyristat enthält.

3. Haarbehandlungsmittel nach einem der Ansprüche 1 oder 2, **dadurch gekennzeichnet, dass** es - bezogen auf sein Gewicht - 0,1 bis 20 Gew.-%, vorzugsweise 0,2 bis 10 Gew.-%, weiter bevorzugt 0,3 bis 7,5 Gew.-%, noch weiter bevorzugt 0,4 bis 5 Gew.-% und insbesondere 0,5 bis 2,5 Gew.-% mindestens einer QAV der Formel I enthält, in der n für die Zahl 20 und A⁻ für Chlorid steht (INCI-Bezeichnung: Behenoyl PG Trimonium Chloride).

4. Haarbehandlungsmittel nach einem der Ansprüche 1 bis 3, **dadurch gekennzeichnet, dass** es - bezogen auf sein Gewicht - 0,1 bis 15 Gew.-%, vorzugsweise 0,2 bis 10 Gew.-%, weiter bevorzugt 0,3 bis 7,5 Gew.-%, noch weiter bevorzugt 0,4 bis 5 Gew.-% und insbesondere 0,5 bis 2,5 Gew.-% mindestens einer QAV der Formel II enthält, in der n für die Zahl 20 und A⁻ für Chlorid steht (INCI-Bezeichnung: Behentrimonium Chloride).

5. Haarbehandlungsmittel nach einem der Ansprüche 1 bis 4, **dadurch gekennzeichnet, dass** es - bezogen auf sein Gewicht - 0,1 bis 15 Gew.-%, vorzugsweise 0,2 bis 10 Gew.-%, weiter bevorzugt 0,3 bis 7,5 Gew.-%, noch weiter bevorzugt 0,4 bis 5 Gew.-% und insbesondere 0,5 bis 2,5 Gew.-% mindestens einer QAV der Formel III enthält, in der R1 und R2 unabhängig voneinander ausgewählt sind aus Stearyl- und Oleoylresten und A - für Methosulfat steht (INCI-Bezeichnung: Quaternium-87).

6. Haarbehandlungsmittel nach einem der Ansprüche 1 bis 5, **dadurch gekennzeichnet, dass** es - bezogen auf sein Gewicht - 0,1 bis 15 Gew.-%, vorzugsweise 0,2 bis 10 Gew.-%, weiter bevorzugt 0,3 bis 7,5 Gew.-%, noch weiter bevorzugt 0,4 bis 5 Gew.-% und insbesondere 0,5 bis 2,5 Gew.-% mindestens eines kationischen Copolymers, enthaltend Monomere der Formel (IV) und Monomere der Formel (V), in denen R¹ = R⁶ = R⁷ = -H und R² = R³ = R⁴ = R⁵ = R⁸ = R⁹ = -CH₃ gilt, n für die Zahl 2 und A- für Chlorid steht (INCI-Bezeichnung: Dimethylacrylamide/Ethyltrimonium Chloride Methacrylate Copolymer) enthält.

7. Haarbehandlungsmittel nach einem der Ansprüche 1 bis 6, **dadurch gekennzeichnet, dass** es - bezogen auf sein Gewicht- 0,1 bis 10 Gew.-%, vorzugsweise 0,15 bis 7,5 Gew.-%, weiter bevorzugt 0,2 bis 5 Gew.-%, noch weiter bevorzugt 0,25 bis 2,5 Gew.-% und insbesondere 0,4 bis 1,5 Gew.-% Polydimethysiloxan (INCl-Bezeichnung: Dimethicone) enthält.

8. Haarbehandlungsmittel nach einem der Ansprüche 1 bis 7, enthaltend bezogen auf sein Gewicht
a) 0,5 bis 2,5 Gew.-% Behenoyl PG Trimonium Chloride,
b) 0,5 bis 2,5 Gew.-% Behentrimonium Chloride,
c) 0,5 bis 2,5 Gew.-% Quaternium-87,
d) 0,5 bis 2,5 Gew.-% Dimethylacrylamide/Ethyltrimonium Chloride Methacrylate Copolymer.

9. Haarbehandlungsmittel nach einem der Ansprüche 1 bis 8, enthaltend bezogen auf sein Gewicht
a) 0,5 bis 2,5 Gew.-% Behenoyl PG Trimonium Chloride,
b) 0,5 bis 2,5 Gew.-% Behentrimonium Chloride),
c) 0,5 bis 2,5 Gew.-% Quaternium-87,
d) 0,5 bis 2,5 Gew.-% Dimethylacrylamide/Ethyltrimonium Chloride Methacrylate Copolymer.
e) 0,5 bis 2,5 Gew.-% Isopropylmyristat.

10. Haarbehandlungsmittel nach einem der Ansprüche 1 bis 9, enthaltend bezogen auf sein Gewicht
a) 0,5 bis 2,5 Gew.-% Behenoyl PG Trimonium Chloride,
b) 0,5 bis 2,5 Gew.-% Behentrimonium Chloride),
c) 0,5 bis 2,5 Gew.-% Quaternium-87,
d) 0,5 bis 2,5 Gew.-% Dimethylacrylamide/Ethyltrimonium Chloride Methacrylate Copolymer.
e) 0,5 bis 2,5 Gew.-% Isopropylmyristat,
f) 0,4 bis 1,5 Gew.-% Dimethicone.

## Claims

1. A hair treatment agent, containing
a) at least one quaternary ammonium compound (QAC) according to formula I 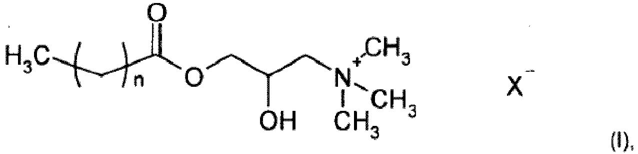 in which n represents an integer from 10 to 24 and X⁻ represents an anion,
b) at least one quaternary ammonium compound (QAC) according to formula II 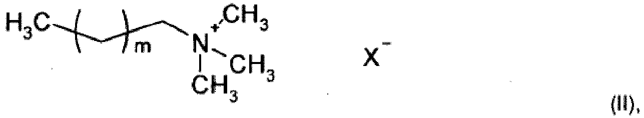 in which m represents an integer from 10 to 24 and X⁻ represents an anion,
c) at least one quaternary ammonium compound (QAC) of formula III 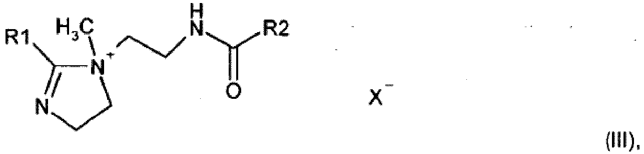 in which R1 and R2 each represent, independently of one another, a saturated or unsaturated, linear or branched hydrocarbon group having a chain length of 16 to 30 carbon atoms and X⁻ represents an anion,
d) at least one cationic copolymer, containing monomers of formula (IV) and monomers of formula (V) 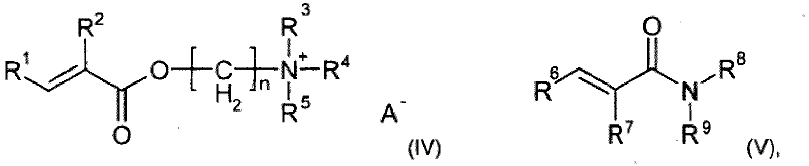 in which R¹ to R⁹ represent, independently of one another, hydrogen, C₁₋₄ alkyl such as methyl, ethyl, propyl, iso-propyl, butyl, iso-butyl or tert-butyl, with the proviso that at least one of the groups R⁶, R⁷, R⁸ or R⁹ represents C₁₋₄ alkyl, n represents integers from 1 to 8, and A⁻ represents a physiologically acceptable anion.

2. The hair treatment agent according to claim 1, **characterized in that** it also contains, based on its weight, 0.1 to 20 wt.%, preferably 0.2 to 10 wt.%, more preferably 0.3 to 7.5 wt.%, even more preferably 0.4 to 5 wt.% and in particular 0.5 to 2.5 wt.%, isopropyl myristate.

3. The hair treatment agent according to one of claims 1 or 2, **characterized in that** it contains, based on its weight, 0.1 to 20 wt.%, preferably 0.2 to 10 wt.%, more preferably 0.3 to 7.5 wt.%, even more preferably 0.4 to 5 wt.% and in particular 0.5 to 2.5 wt.%, of at least one QAC of formula I, in which n represents the number 20 and A⁻ represents chloride (INCl name: Behenoyl PG-Trimonium Chloride).

4. The hair treatment agent according to one of claims 1 to 3, **characterized in that** it contains, based on its weight, 0.1 to 15 wt.%, preferably 0.2 to 10 wt.%, more preferably 0.3 to 7.5 wt.%, even more preferably 0.4 to 5 wt.% and in particular 0.5 to 2.5 wt.%, of at least one QAC of formula II, in which n represents the number 20 and A- represents chloride (INCl name: Behentrimonium Chloride).

5. The hair treatment agent according to one of claims 1 to 4, **characterized in that** it contains, based on its weight, 0.1 to 15 wt.%, preferably 0.2 to 10 wt.%, more preferably 0.3 to 7.5 wt.%, even more preferably 0.4 to 5 wt.% and in particular 0.5 to 2.5 wt.%, of at least one QAC of formula III, in which R1 and R2 are selected, independently of one another, from stearyl and oleoyl groups and A⁻ represents methosulfate (INCl name: Quaternium-87).

6. The hair treatment agent according to one of claims 1 to 5, **characterized in that** it contains, based on its weight, 0.1 to 15 wt.%, preferably 0.2 to 10 wt.%, more preferably 0.3 to 7.5 wt.%, even more preferably 0.4 to 5 wt.% and in particular 0.5 to 2.5 wt.%, of at least one cationic copolymer, containing monomers of formula (IV) and monomers of formula (V), in which R¹ = R⁶ = R⁷ = -H and R² = R³ = R⁴ = R⁵ = R⁸ = R⁹ = -CH₃, n represents the number 2 and A⁻ represents chloride (INCl name: Dimethylacrylamide/Ethyltrimonium Chloride Methacrylate Copolymer).

7. The hair treatment agent according to one of claims 1 to 6, **characterized in that** it contains, based on its weight, 0.1 to 10 wt.%, preferably 0.15 to 7.5 wt.%, more preferably 0.2 to 5 wt.%, even more preferably 0.25 to 2.5 wt.%, and in particular 0.4 to 1.5 wt.%, polydimethylsiloxane (INCI name: Dimethicone).

8. The hair treatment agent according to one of claims 1 to 7, containing, based on its weight,
a) from 0.5 to 2.5 wt.% Behenoyl PG-Trimonium Chloride,
b) from 0.5 to 2.5 wt.% Behentrimonium Chloride,
c) from 0.5 to 2.5 wt.% Quaternium-87,
d) from 0.5 to 2.5 wt.% Dimethylacrylamide/Ethyltrimonium Chloride Methacrylate Copolymer.

9. The hair treatment agent according to one of claims 1 to 8, containing, based on its weight,
a) from 0.5 to 2.5 wt.% Behenoyl PG-Trimonium Chloride,
b) from 0.5 to 2.5 wt.% Behentrimonium Chloride,
c) from 0.5 to 2.5 wt.% Quaternium-87,
d) from 0.5 to 2.5 wt.% Dimethylacrylamide/Ethyltrimonium Chloride Methacrylate Copolymer.
e) from 0.5 to 2.5 wt.% isopropyl myristate.

10. The hair treatment agent according to one of claims 1 to 9, containing, based on its weight,
a) from 0.5 to 2.5 wt.% Behenoyl PG-Trimonium Chloride,
b) from 0.5 to 2.5 wt.% Behentrimonium Chloride,
c) from 0.5 to 2.5 wt.% Quaternium-87,
d) from 0.5 to 2.5 wt.% Dimethylacrylamide/Ethyltrimonium Chloride Methacrylate Copolymer.
e) from 0.5 to 2.5 wt.% isopropyl myristate,
f) from 0.4 to 1.5 wt.% Dimethicone.

## Revendications

1. Agent de traitement capillaire contenant
a) au moins un composé d'ammonium quaternaire (QAV) selon la formule I 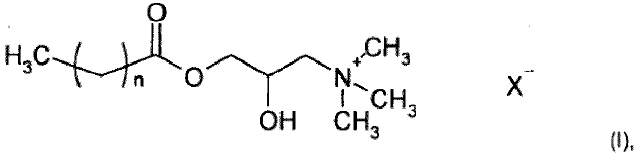 dans laquelle n représente un nombre entier valant de 10 à 24 et X⁻ représente un anion,
b) au moins un composé d'ammonium quaternaire (QAV) selon la formule II 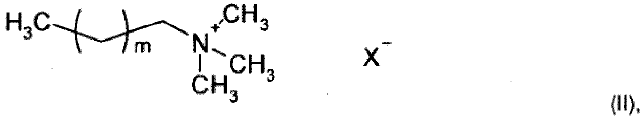 dans laquelle m représente un nombre entier valant de 10 à 24 et X⁻ représente un anion,
c) au moins un composé d'ammonium quaternaire (QAV) selon la formule III 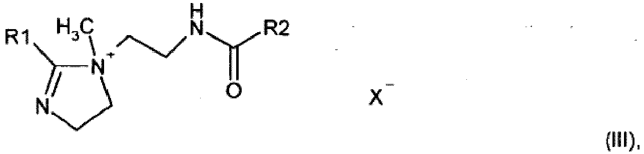 dans laquelle R1 et R2 représentent respectivement, indépendamment l'un de l'autre, un radical hydrocarbure saturé ou insaturé, linéaire ou ramifié, ayant une longueur de chaîne allant de 16 à 30 atomes de carbone et X⁻ représente un anion,
d) au moins un copolymère cationique contenant des monomères de la formule (IV) et des monomères de la formule (V) 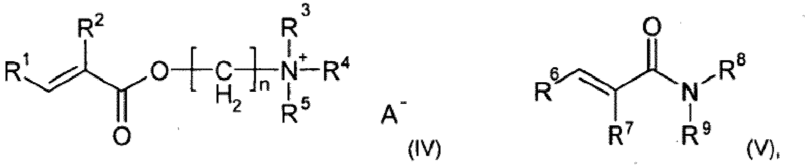 dans lesquels R¹ à R⁹ représentent indépendamment les uns des autresl'hydrogène, un alkyle en C₁-C₄ tel que le méthyle, l'éthyle, le propyle, l'isopropyle, le butyle, l'isobutyle ou le tert-butyle, à condition qu'au moins un des radicaux R⁶, R⁷, R⁸ ou R⁹ représente un groupe alkyle en C₁-C₄, n représente un nombre entier valant de 1 à 8, et A⁻ représente un anion physiologiquement compatible.

2. Agent de traitement capillaire selon la revendication 1, **caractérisée en ce qu'**il contient - rapporté à son poids - en outre de 0,1 à 20 % en poids, de préférence de 0,2 à 10 % en poids, de manière davantage préférée de 0,3 à 7,5 % en poids, de manière encore davantage préférée de 0,4 à 5 % en poids et en particulier de 0,5 à 2,5 % en poids de myristate d'isopropyle.

3. Agent de traitement capillaire selon l'une des revendications 1 ou 2, **caractérisé en ce qu'**il contient - rapporté à son poids - de 0,1 à 20 % en poids, de préférence de 0,2 à 10 % en poids, de manière davantage préférée de 0,3 à 7,5 % en poids, de manière encore davantage préférée de 0,4 à 5 % en poids et en particulier de 0,5 à 2,5 % en poids d'au moins un QAV de la formule I, dans laquelle n représente le nombre 20 et A⁻ représente le chlorure (dénomination INCl : Behenoyl PG Trimonium Chloride).

4. Agent de traitement capillaire selon l'une des revendications 1 à 3, **caractérisé en ce qu'**il contient - rapporté à son poids - de 0,1 à 15 % en poids, de préférence de 0,2 à 10 % en poids, de manière davantage préférée de 0,3 à 7,5 % en poids, de manière encore davantage préférée de 0,4 à 5 % en poids et en particulier de 0,5 à 2,5 % en poids d'au moins un QAV de la formule II, dans laquelle n représente le nombre 20 et A⁻ représente le chlorure (dénomination INCl : Behentrimonium Chloride).

5. Agent de traitement capillaire selon l'une des revendications 1 à 4, **caractérisé en ce qu'**il contient - rapporté à son poids - de 0,1 à 15 % en poids, de préférence de 0,2 à 10 % en poids, de manière davantage préférée de 0,3 à 7,5 % en poids, de manière encore davantage préférée de 0,4 à 5 % en poids et en particulier de 0,5 à 2,5 % en poids d'au moins un QAV de formule III, dans laquelle R1 et R2 sont sélectionnés indépendamment l'un de l'autre parmi les radicaux de stéaryle et d'oléoyle et A⁻ représente le méthosulfate (dénomination INCl : Quaternium- 87).

6. Agent de traitement capillaire selon l'une des revendications 1 à 5, **caractérisé en ce qu'**il contient - rapporté à son poids - de 0,1 à 15 % en poids, de préférence de 0,2 à 10 % en poids, de manière davantage préférée de 0,3 à 7,5 % en poids, de manière encore davantage préférée de 0,4 à 5 % en poids et en particulier de 0,5 à 2,5 % en poids d'au moins un copolymère cationique, contenant des monomères de formule (IV) et des monomères de formule (V), dans lesquels R¹ = R⁶ = R⁷ = -H et R² = R³ = R⁴ = R⁵ = R⁸ = R⁹ = -CH₃, n représente le chiffre 2 et A⁻ représente le chlorure (dénomination INCl : Dimethylacrylamide/Ethyltrimonium Chloride Methacrylate Copolymer).

7. Agent de traitement capillaire selon l'une des revendications 1 à 6, **caractérisé en ce qu'**il contient - rapporté à son poids - de 0,1 à 10 % en poids, de préférence de 0,15 à 7,5 % en poids, de manière davantage préférée de 0,2 à 5 % en poids, de manière encore davantage préférée de 0,25 à 2,5 % en poids et en particulier de 0,4 à 1,5 % en poids de polydiméthylsiloxane (dénomination INCl : Dimethicone).

8. Agent de traitement capillaire selon l'une des revendications 1 à 7, contenant - rapporté à son poids
a) de 0,5 à 2,5 % en poids de Behenoyl PG Trimonium Chloride,
b) de 0,5 à 2,5 % en poids de Behentrimonium Chloride,
c) de 0,5 à 2,5 % en poids de Quaternium-87,
d) de 0,5 à 2,5 % en poids de Dimethylacrylamide/Ethyltrimonium Chloride Methacrylate Copolymer.

9. Agent de traitement capillaire selon l'une des revendications 1 à 8, contenant - rapporté à son poids
a) de 0,5 à 2,5 % en poids de Behenoyl PG Trimonium Chloride,
b) de 0,5 à 2,5 % en poids de Behentrimonium Chloride,
c) de 0,5 à 2,5 % en poids de Quaternium-87,
d) de 0,5 à 2,5 % en poids de Dimethylacrylamide/Ethyltrimonium Chloride Methacrylate Copolymer.
e) de 0,5 à 2,5 % en poids de myristate d'isopropyle.

10. Agent de traitement capillaire selon l'une des revendications 1 à 9, contenant - rapporté à son poids
a) de 0,5 à 2,5 % en poids de Behenoyl PG Trimonium Chloride,
b) de 0,5 à 2,5 % en poids de Behentrimonium Chloride,
c) de 0,5 à 2,5 % en poids de Quaternium-87,
d) de 0,5 à 2,5 % en poids de Dimethylacrylamide/Ethyltrimonium Chloride Methacrylate Copolymer.
e) de 0,5 à 2,5 % en poids de myristate d'isopropyle,
f) de 0,4 à 1,5 % en poids de Dimethicone.
